# FASCICULE DE BREVET EUROPEEN

(11) **EP 1 366 747 B1**
(45) Date de publication et mention de la délivrance du brevet: **22.07.2009**
(21) Numéro de dépôt: 03291139.8
(22) Date de dépôt: 15.05.2003
(51) Int. Cl.: A61K 8/00, A61K 8/02, A61K 8/04, A61K 8/30, A61K 8/89, A61Q 5/06, B65D 83/62

(54) **Dispositif aérosol à deux compartiments comprenant une composition de coiffage, alcoolique ou hydroalcoolique, et procédé de coiffage**
Zweikammeraerosolvorrichtung die eine alkoholische oder wässrig-alkoholische Frisierzusammensetzung enthält und Frisierverfahren
Two-compartment aerosol can comprising an alcoholic or hydroalcoholic hair dressing composition and hair dressing process

(30) Priorité: 31.05.2002 FR 0206733
(43) Date de publication de la demande: 03.12.2003
(73) Titulaire: L'OREAL, S.A., 75008 Paris (FR)
(72) Inventeur: Le Bourhis, François, 93300 Aubervilliers (FR)
(74) Mandataire: Bourdeau, Françoise

(56) Documents cités:
- WO-A-02/51364
- FR-A- 2 815 853
- US-A- 3 966 087
- US-A- 6 083 494
- US-B1- 6 346 234

## Description

La présente invention est relative à un dispositif aérosol à deux compartiments comprenant une composition de coiffage et un gaz comprimé particulier, et à un procédé de coiffage.

Les compositions de coiffage, telles que les laques et les sprays, conditionnées sous forme de spray aérosol sont généralement composées d'une phase liquide comprenant, dans un milieu cosmétiquement acceptable aqueux, alcoolique ou hydroalcoolique, au moins un polymère de fixation, et d'un agent propulseur qui est un gaz liquéfié sous pression réduite ou dissous dans la phase liquide.

D'autres dispositifs aérosols comprenant une telle phase liquide existent, mais ils comprennent des gaz comprimés pour assurer la propulsion de la phase liquide. Or ces dispositifs à gaz comprimés présentent l'inconvénient d'une perte de pression des gaz au cours du temps. En effet, on observe une fuite des gaz comprimés au cours du temps ou lors d'une mauvaise utilisation du récipient, c'est-à-dire lorsqu'il se retrouve la tête en bas.

Cet inconvénient entraîne une propulsion de moins en moins bonne de la phase liquide au cours du temps et par conséquent une répartition de moins en moins bonne de la phase liquide sur les cheveux.

Un autre facteur pouvant conduire à une mauvaise répartition de la phase liquide à partir de ces dispositifs est la qualité du spray qui se dégrade lorsque l'on cherche à obtenir une fixation la plus importante possible, c'est-à-dire lorsque l'on cherche à augmenter la quantité de polymère que l'on peut dissoudre dans ladite phase liquide.

La demanderesse a fait la découverte surprenante que la séparation par voie mécanique de la phase liquide du gaz comprimé assurant la propulsion, et l'utilisation d'un gaz comprimé particulier et d'un polyuréthane fixant permettait de résoudre les problèmes de fuite et de répartition de la phase liquide sur les cheveux. Cette combinaison particulière permet ainsi d'obtenir une fixation et/ou une mise en forme avec un niveau de fixation qui ne peut être atteint habituellement avec ce type de produit.

L'invention a donc pour objet un dispositif aérosol à deux compartiments comprenant une composition de coiffage qui comprend au moins un polyuréthane fixant, et un gaz comprimé tel que décrit ci-dessous.

Un autre objet de la présente invention consiste en un procédé de coiffage.

D'autres objets, caractéristiques, aspects et avantages de l'invention apparaîtront encore plus clairement à la lecture de la description et des divers exemples qui suivent.

Le dispositif aérosol à deux compartiments selon l'invention comprend :
(a) dans un premier compartiment, une composition de coiffage comprenant au moins un polyuréthane fixant dans un milieu cosmétiquement acceptable alcoolique ou hydroalcoolique qui comprend au moins 5 % en poids, par rapport au poids total de ladite composition, d'un alcool monohydrique en C₁-C₄, et
(b) dans un deuxième compartiment, un gaz comprimé choisi parmi l'air, l'azote, le gaz carbonique et leurs mélanges, l'air étant particulièrement préféré.

Ledit gaz comprimé est utilisé de préférence sous une pression comprise entre 1 et 12 bars, mieux encore comprise entre 9 et 11 bars.

Par polyuréthane fixant, on entend au sens de la présente invention, des polycondensats comprenant au moins une séquence polyuréthanne susceptible d'apporter du maintien à la coiffure. Ils sont décrits en particulier dans les brevets EP 0 751 162, EP 0 637 600, FR 2 743 297 et EP 0 648 485 dont la Demanderesse est titulaire, ainsi que les brevets EP 0 656 021 ou WO 94/03510 de la Société BASF et EP 0 619 111 de la Société National Starch.

Les polyuréthanes fixants utilisés conformément à l'invention peuvent être solubles dans le milieu cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polyuréthane fixant.

Selon l'invention, on peut utiliser tout type de tensioactif dans ladite dispersion, mais de préférence un tensioactif non ionique. La taille moyenne des particules du polyuréthane fixant dans la dispersion est de préférence comprise entre 0,1 et 1 micromètre.

A titre d'exemple, le polyuréthane fixant peut être formé par un arrangement de blocs, cet arrangement étant obtenu notamment à partir de :
(1) au moins un composé qui contient deux ou plus de deux atomes d'hydrogène actifs par molécule ;
(2) au moins un diol ou un mélange de diols contenant des fonctions acides ou leurs sels ; et
(3) au moins un di- ou polyisocyanate.

Avantageusement, les composés (1) sont choisis dans le groupe comprenant les diols, les diamines, les polyesterols, les polyétherols ou leurs mélanges.

Les composés (1) qui sont préférés sont les polyéthylèneglycols et les polypropylèneglycols linéaires, en particulier ceux qui sont obtenus par réaction de l'oxyde d'éthylène ou de propylène avec l'eau ou du diéthylène ou du dipropylèneglycol en présence d'hydroxyde de sodium en tant que catalyseur. Ces polyalkylèneglycols ont généralement une masse moléculaire comprise entre environ 600 et 20 000.

D'autres composés organiques préférés sont ceux qui ont des groupes mercapto, amino, carboxyle ou hydroxyle. Parmi ceux-ci, on cite plus particulièrement les composés polyhydroxylés tels que les polyéther-diols, les polyester-diols, les polyacétal-diols, les polyamide-diols, les polyester-polyamide-diols, les poly(alkylène éther)-diols, les polythioéther-diols et les polycarbonate-diols.

Les polyéther-diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéthers appropriés sont par exemple préparés par condensation d'oxydes d'alkylène et d'alcools polyhydriques, tels que l'éthylèneglycol, le 1,2-propylèneglycol et le 1,4-butanediol.

Les polyester-diols, polyester-amides, polyamide-diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylèneglycol, le 1,2-propylèneglycol, le 1,4-butanediol, le néopentylglycol et l'hexanediol. On peut aussi utiliser des aminoalcools, par exemple l'éthanolamine. Des diamines appropriées pour préparer les amide-polyesters sont l'éthylène-diamine et l'hexaméthylène-diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycols seuls ou en combinaison avec d'autres glycols tels que l'éthylèneglycol, le 1,2-propylèneglycol ou avec d'autres composés polyhydroxylés. Les composés polyhydroxylés contenant déjà des groupements uréthannes, des polyols naturels, qui peuvent être davantage modifiés, par exemple, l'huile de castor et les carbohydrates peuvent également être utilisés.

Plus préférentiellement, le composé du groupe (1) est un polyestérol, notamment un polyester-diol formé par la réaction d'au moins un (di)-polyol (1ₐ) et d'au moins un acide (1_{b}). Le (di)- polyol (1ₐ) est en particulier choisi dans le groupe comprenant le néopentylglycol, le 1,4-butanediol, l'hexanediol, l'éthylèneglycol, le diéthylèneglycol, le propylèneglycol, le butylèneglycol, le néopentylglycol et (di)-polyéthylèneglycol. L'acide (1_{b}) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol-propanoïque (DMPA) ou un acide 2,2-hydroxyméthyl-carboxylique. En général, le composé (2) est utile en tant que bloc de couplage. En tant que composés (2), on préfère ceux comprenant au moins un poly (acide-(alpha-hydroxycarboxyliquediol)).

Les composés (2) particulièrement préférés conformément à l'invention sont ceux choisis dans le groupe comprenant l'acide 2,2-di-(hydroxyméthyl)acétique, l'acide 2,2-dihydroxyméthylpropionique, l'acide 2,2-dihydroxyméthylbutyrique, l'acide 2,2-dihydroxyméthyl-pentanoïque.

Le di- ou polyisocyanate (3) peut être choisi en particulier dans le groupe comprenant l'hexaméthylène-diisocyanate, l'isophorone-diisocyanate (IDPI), le toluylène-diisocyanate, le diphénylméthane-4,4'-diisocyanate (DPMD) et le dicyclohexylméthane-4,4'-diisocyanate (DCMD), le méthylène-di-p-phényl-diisocyanate, le méthylène-bis(4-cyclohexylisocyanate), les toluène-diisocyanates, le 1,5-naphtalène-diisocyanate, le 4,4'-diphénylméthane-diisocyanate, le 2,2'-diméthyl-4,4'-diphénylméthane-diisocyanate, le 1,3-phénylène-diisocyanate, le 1,4-phénylène diisocyanate, des mélanges de 2,4- et de 2,6-toluène-diisocyanates, le 2,2'-dichloro-4,4'-diisocyanato-diphénylméthane, le 2,4-dibromo-1,5-diisocyanato naphtalène, le butane-1,4-diisocyanate, l'hexane-1,6-diisocyanate, le cyclohexane-1,4-diisocyanate.

Le polyuréthane fixant peut être formé à l'aide d'un composé supplémentaire (4) servant en général à allonger sa chaîne. Ces composés (4) peuvent être choisis dans le groupe comprenant notamment les glycols saturés ou insaturés tels que l'éthylèneglycol, le diéthylèneglycol, le néopentylglycol, le triéthylène glycol ; les aminoalcools tels que l'éthanolamine, la propanolamine, la butanolamine ; les amines primaires hétérocycliques, aromatiques, cycloaliphatiques, et aliphatiques ; les diamines ; les acides carboxyliques tels que les acides carboxyliques aliphatiques, aromatiques et hétérocycliques comme les acides oxalique, succinique, glutarique, adipique, sébacique et téréphtalique ; les acides aminocarboxyliques. Les composés (4) préférés sont les diols aliphatiques.

Les polyuréthanes fixants utilisés selon l'invention peuvent également être formés à partir de composés supplémentaires (5) ayant un squelette siliconé tels que les polysiloxanes, les polyalkylsiloxanes ou les polyarylsiloxanes, notamment les polyéthylsiloxanes, les polyméthylsiloxanes et les polyphénylsiloxanes, comportant éventuellement des chaînes hydrocarbonées greffées sur les atomes de silicium.

Les polyuréthanes fixants utilisés avantageusement comprennent un motif répétitif de base répondant à la formule générale (I) :

-O-B-O-CO-NH-R-NH-CO- (I)

dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes alkylènes de type aromatique, aliphatique en C₁ à C₂₀, cycloaliphatique en C₁ à C₂₀, ces groupes étant substitués ou non.

Le groupe R est avantageusement choisi parmi les groupes répondant aux formules suivantes :

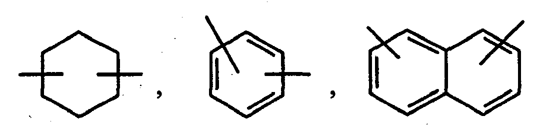

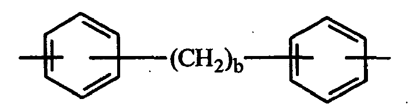

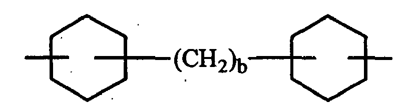

-(CH₂)_{c}-

dans lesquelles b est un nombre entier compris entre 0 et 3, et c un nombre entier compris entre 1 et 20, de préférence compris entre 2 et 12.

En particulier, le groupe R est choisi parmi les groupes hexaméthylène, 4,4'-biphénylèneméthane, 2,4- et/ou 2,6-tolylène, 1,5-naphtylène, p-phénylène, méthylène- 4,4-bis-cyclohexyle et le groupe divalent dérivé de l'isophorone.

Le polyuréthane fixant utilisé dans la présente invention peut avantageusement comprendre en outre au moins une séquence polysiloxane et son motif répétitif de base répond par exemple à la formule générale (II) :

-O-P-O-CO-NH-R-NH-CO- (II)

dans laquelle :
- P est un segment polysiloxanique, et
- R est un groupe divalent choisi parmi les groupes alkylènes de type aromatique, aliphatique en C₁ à C₂₀, cycloaliphatique en C₁ à C₂₀, ces groupes étant substitués ou non.

Avantageusement, le segment polysiloxanique P répond à la formule générale (III) ci-après : 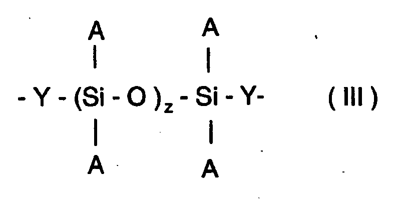 dans laquelle:
- les groupes A, qui peuvent être identiques ou différents, sont choisis parmi d'une part les groupes hydrocarbonés monovalents en C₁ à C₂₀ exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne du segment polysiloxane soit comprise entre 300 et 10 000.

En général, le groupe divalent Y est choisi parmi les groupes alkylènes de formule -(CH₂)ₐ- , dans laquelle a représente un nombre entier pouvant être compris entre 1 et 10.

Les groupes A peuvent être choisis parmi les groupes alkyles, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle ; les groupes arylalkyle, notamment benzyle et phényléthyle, ainsi que les groupes tolyle et xylyle.

A titre d'exemples de polyuréthane fixant, on peut notamment citer le copolymère acide diméthylolpropionique/isophorone-diisocyanate/néopentylglycol/polyesterdiols (connu aussi sous le nom de polyuréthane-1, appellation INCI) vendu sous la marque Luviset^{®} PUR par la société BASF, le copolymère acide diméthylol-propionique/isophorone-diisocyanate/néopentylglycol/polyesterdiols/ diamine siliconée (connu aussi sous le nom de polyuréthane-6, appellation INCI) vendu sous la marque Luviset^{®} Si PUR A par la société BASF.

Ledit polyuréthane fixant est de préférence présent en une quantité comprise entre 0,5 et 20 % en poids, mieux encore entre 1 et 12 % en poids par rapport au poids total de la composition de coiffage.

Par milieu cosmétiquement acceptable, on entend un milieu compatible avec les cheveux, mais aussi d'odeur, d'aspect et de toucher agréables.

Le milieu cosmétiquement acceptable alcoolique ou hydroalcoolique peut être constitué par au moins un alcool tel qu'un alcool en C₁₋₄, par exemple l'éthanol ou l'isopropanol, ou par un mélange d'eau et d'au moins un alcool, comme un alcool en C₁₋₄.

Le milieu comprend au moins 5 % en poids, de préférence de 5 à 99,9 % en poids, mieux encore de 10 à 99 % en poids et encore plus préférentiellement de 20 à 98 % en poids par rapport au poids total de ladite composition, d'un alcool monohydrique en C₁₋₄.

Dans un milieu hydroalcoolique, la proportion d'eau peut varier entre 1 et 95 %, et de préférence entre 10 à 90 % du poids total de la composition.

La composition de coiffage peut comprendre en outre des additifs tels que des silicones sous forme soluble, dispersée, micro-dispersée, des polymères fixants non polyuréthane, non ioniques, cationiques, anioniques ou amphotères, des actifs traitants, des agents hydratants comme le glycérol, des filtres UV, des acides, des bases, des agents plastifiants, des agents solubilisants, des agents conservateurs, des vitamines et des provitamines, des colorants, des pigments, des agents tensioactifs anioniques, cationiques, non ioniques ou amphotères, des parfums, des agents anti-corrosion, et leurs mélanges.

L'homme de métier veillera à choisir les éventuels additifs et leur quantité de manière à ce qu'ils ne nuisent pas aux propriétés des compositions de la présente invention.

Ces additifs sont notamment présents dans la composition selon l'invention en une quantité allant de 0 à 20 % en poids par rapport au poids total de la composition.

De préférence, le dispositif aérosol à deux compartiments est constitué par un bidon aérosol externe comportant une poche interne soudée hermétiquement à une valve. La composition est introduite dans la poche interne et un gaz comprimé est introduit entre la poche et le bidon à une pression suffisante pour faire sortir le produit sous forme d'un spray à travers l'orifice d'une buse. Un tel dispositif est commercialisé sous le nom EP SPRAY par la société EP-SPRAY SYSTEM SA.

Les dispositifs aérosols de l'invention sont de préférence des laques pour cheveux.

La présente invention concerne également un procédé de coiffage qui consiste en ce que l'on vaporise la composition de coiffage contenue dans le dispositif aérosol selon l'invention, sur les cheveux mouillés ou non.

Les exemples suivants sont donnés à titre illustratif de la présente invention.

Dans les exemples suivants, toutes les quantités sont indiquées en pour cent en poids par rapport au poids total de la composition, sauf indication contraire.

### Exemple 1

On a préparé une composition de coiffage à partir des ingrédients suivants :

| | | % en poids |
|---|---|---|
| Copolymère acide diméthylolpropionique/isophorone-diisocyanate/néopentylglycol/polyesterdiols/diamine siliconée vendu sous le nom LUVISET^{®} SI PUR A par la société BASF, à 30 % en matière active dans un mélange eau/alcool 60/10 | | 30,00 |
| Méthoxycinnamate d'éthylhexyle vendu sous la dénomination commerciale PARSOL^{®} MCX par la société ROCHE VITAMINS | | 0,05 |
| Parfum | | 0,10 |
| Alcool éthylique absolu qsp | | 100 |

On a introduit la composition préparée ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonaire.

La poche est remplie avec la composition comme indiqué ci-dessus. De l'air comprimé est introduit entre la poche et le bidon.

On a vaporisé la composition sur des cheveux secs. La pulvérisation se fait sous forme d'un spray doux.

On obtient, après séchage, une chevelure présentant un bon maintien.

### Exemple 2

On a préparé une deuxième composition de coiffage à partir des ingrédients suivants :

| | | % en poids |
|---|---|---|
| Copolymère acide diméthylolpropionique/isophorone-diisocyanate/néopentylglycol/polyesterdiols/diamine siliconée vendu sous le nom LUVISET^{®} SI-PUR A par la société BASF, à 30 % en matière active dans un mélange eau/alcool 60/10 | | 30,00 |
| Méthoxycinnamate d'éthylhexyle vendu sous la dénomination commerciale PARSOL^{®} MCX par la société ROCHE VITAMINS | | 0,05 |
| Eau déminéralisée | | 18 |
| Parfum | | 0,10 |
| Alcool éthylique absolu qsp | | 100 |

On a introduit la composition préparée ci-dessus dans un dispositif de distribution aérosol commercialisé sous le nom EP SPRAY tel que décrit dans l'exemple 1.

On a vaporisé la composition sur des cheveux secs.

On obtient des résultats proches de ceux obtenus dans le cas de l'exemple 1.

## Revendications

1. Dispositif aérosol à deux compartiments, comprenant :
a) dans un premier compartiment, une composition de coiffage comprenant au moins un polyuréthane fixant dans un milieu cosmétiquement acceptable alcoolique ou hydroalcoolique qui comprend au moins 5 % en poids par rapport au poids de ladite composition, d'un alcool monohydrique en C₁-C₄, et
b) dans un deuxième compartiment, un gaz comprimé choisi parmi l'air, l'azote, le gaz carbonique et leurs mélanges.

2. Dispositif aérosol selon la revendication 1, **caractérisé en ce que** le gaz comprimé est l'air.

3. Dispositif aérosol selon l'une quelconque des revendications 1 ou 2 **caractérisé en ce que** la pression du gaz comprimé est comprise entre 1 et 12 bars.

4. Dispositif aérosol selon la revendication 3, **caractérisé en ce que** la pression du gaz comprimé est comprise entre 9 et 11 bars.

5. Dispositif aérosol selon l'une quelconque des revendications précédentes, **caractérisé en ce que** le polyuréthane fixant comprend un motif répétitif de base répondant à la formule générale (I) :
-O-B-O-CO-NH-R-NH-CO- (I)
dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes alkylènes de type aromatique, aliphatique en C₁ à C₂₀, cycloaliphatique en C₁ à C₂₀, ces groupes étant substitués ou non.

6. Dispositif aérosol selon l'une quelconque des revendications 1 à 5, **caractérisée en ce que** le polyuréthane fixant comprend un motif répétitif de base répondant à la formule générale (II) :
-O-P-O-CO-NH-R-NH-CO- (II)
dans laquelle :
- P est un segment polysiloxanique, et
- R est un groupe divalent choisi parmi les groupes alkylènes de type aromatique, aliphatique en C₁ à C₂₀, cycloaliphatique en C₁ à C₂₀, ces groupes étant substitués ou non.

7. Dispositif aérosol selon la revendication 5 ou 6, **caractérisée en ce que** le polyuréthane fixant est un copolymère acide diméthylol-propionique/isophorone-diisocyanate/néopentylglycol/ polyester-diols/diamine siliconée.

8. Dispositif aérosol selon l'une quelconque des revendications précédentes, **caractérisé en ce que** le polyuréthane fixant est présent en une quantité comprise entre 0,5 et 20 % en poids par rapport au poids total de la composition.

9. Dispositif aérosol selon la revendication 8, **caractérisé en ce que** le polyuréthane fixant est présent en une quantité comprise entre 1 et 12 % en poids par rapport au poids total de la composition.

10. Dispositif selon l'une quelconque des revendications précédentes, **caractérisé en ce que** le milieu cosmétiquement acceptable alcoolique ou hydroalcoolique est constitué par au moins un alcool ou par un mélange d'au moins un alcool et d'eau.

11. Dispositif aérosol selon la revendication 10, **caractérisé en ce que** l'alcool est un alcool en C₁₋₄.

12. Dispositif aérosol selon la revendication 11, **caractérisé en ce que** l'alcool est l'éthanol ou l'isopropanol.

13. Dispositif aérosol selon l'une quelconque des revendications précédentes, **caractérisé en ce que** la composition de coiffage comprend en outre des additifs choisis parmi les silicones sous forme soluble, dispersée, micro-dispersée, les polymères fixants non polyuréthanes, non ioniques, cationiques, anioniques ou amphotères, des actifs traitants, des agents hydratants, des filtres UV, des acides, des bases, des agents plastifiants, des agents solubilisants, des agents conservateurs, des vitamines et des provitamines, des colorants, des pigments, des agents tensioactifs anioniques, cationiques, non ioniques ou amphotères, des parfums, des agents anti-corrosion, et leurs mélanges.

14. Dispositif aérosol selon l'une quelconque des revendications précédentes, **caractérisé en ce qu'**il constitue une laque pour cheveux.

15. Procédé de coiffage, **caractérisé en ce que** l'on vaporise la composition de coiffage contenue dans le dispositif aérosol selon l'une quelconque des revendications précédentes, sur les cheveux mouillés ou non.

## Claims

1. Two-compartment aerosol device comprising:
(a) in a first compartment, a styling composition comprising at least one fixing polyurethane in an alcoholic or aqueous-alcoholic cosmetically acceptable medium that comprises at least 5% by weight, relative to the weight of the said composition, of a C₁-C₄ monohydric alcohol, and
(b) in a second compartment, a compressed gas chosen from air, nitrogen and carbon dioxide, and mixtures thereof.

2. Aerosol device according to Claim 1, **characterized in that** the compressed gas is air.

3. Aerosol device according to either of Claims 1 and 2, **characterized in that** the pressure of the compressed gas is between 1 and 12 bar.

4. Aerosol device according to Claim 3, **characterized in that** the pressure of the compressed gas is between 9 and 11 bar.

5. Aerosol device according to any one of the preceding claims, **characterized in that** the fixing polyurethane comprises a base repeating unit corresponding to the general formula (I):
-O-B-O-CO-NH-R-NH-CO- (I)
in which:
- B is a divalent C₁ to C₃₀ hydrocarbon-based group, this group being optionally substituted with a group comprising one or more carboxylic acid functions and/or one or more sulfonic acid functions, the said carboxylic acid and/or sulfonic acid functions being in free form or partially or totally neutralized with a mineral or organic base, and
- R is a divalent group chosen from alkylene groups of aromatic type, C₁ to C₂₀ aliphatic groups and C₁ to C₂₀ cycloaliphatic groups, these groups being substituted or unsubstituted.

6. Aerosol device according to any one of Claims 1 to 5, **characterized in that** the fixing polyurethane comprises a base repeating unit corresponding to the general formula (II):
-O-P-O-CO-NH-R-NH-CO- (II)
in which:
- P is a polysiloxane segment, and
- R is a divalent group chosen from alkylene groups of aromatic type, C₁ to C₂₀ aliphatic groups and C₁ to C₂₀ cycloaliphatic groups, these groups being substituted or unsubstituted.

7. Aerosol device according to Claim 5 or 6, **characterized in that** the fixing polyurethane is a dimethylolpropionic acid/isophorone diisocyanate/ neopentyl glycol/polyesterdiols/silicone diamine copolymer.

8. Aerosol device according to any one of the preceding claims, **characterized in that** the fixing polyurethane is present in an amount of between 0.5% and 20% by weight relative to the total weight of the composition.

9. Aerosol device according to Claim 8, **characterized in that** the fixing polyurethane is present in an amount of between 1% and 12% by weight relative to the total weight of the composition.

10. Device according to any one of the preceding claims, **characterized in that** the alcoholic or aqueous-alcoholic cosmetically acceptable medium consists of at least one alcohol or of a mixture of at least one alcohol and water.

11. Aerosol device according to Claim 10, **characterized in that** the alcohol is a C₁₋₄ alcohol.

12. Aerosol device according to Claim 11, **characterized in that** the alcohol is ethanol or isopropanol.

13. Aerosol device according to any one of the preceding claims, **characterized in that** the styling composition also comprises additives chosen from silicones in soluble, dispersed or microdispersed form, nonionic, cationic, anionic or amphoteric non-polyurethane fixing polymers, treating active agents, moisturizers, UV-screening agents, acids, bases, plasticizers, solubilizers, preserving agents, vitamins and provitamins, colorants, pigments, anionic, cationic, nonionic or amphoteric surfactants, fragrances and anticorrosion agents, and mixtures thereof.

14. Aerosol device according to any one of the preceding claims, **characterized in that** it constitutes a hair lacquer.

15. Styling process, **characterized in that** the styling composition contained in the aerosol device according to any one of the preceding claims is vaporized onto wet or dry hair.

## Patentansprüche

1. Aerosolvorrichtung mit zwei Compartments, die aufweist:
(a) in einem ersten Compartment eine Frisierzusammensetzung, die mindestens ein fixierendes Polyurethan in einem kosmetisch akzeptablen alkoholischen oder wässerigalkoholischen Medium enthält, das mindestens 5 Gew.-% eines einwertigen C₁-C₄-Alkohols, bezogen auf das Gewicht der Zusammensetzung, enthält,
und
(b) in einem zweiten Compartment ein komprimiertes Gas, das ausgewählt ist unter Luft, Stickstoff und Kohlendioxid-Gas und Gemischen davon.

2. Aerosolvorrichtung nach Anspruch 1, **dadurch gekennzeichnet, dass** das komprimierte Gas Luft ist.

3. Aerosolvorrichtung nach Anspruch 1 oder 2, **dadurch gekennzeichnet, dass** der Druck des komprimierten Gases im Bereich von 1 bis 12 bar liegt.

4. Aerosolvorrichtung nach Anspruch 3, **dadurch gekennzeichnet, dass** der Druck des komprimierten Gases im Bereich von 9 bis 11 bar liegt.

5. Aerosolvorrichtung nach einem der vorhergehenden Ansprüche, **dadurch gekennzeichnet, dass** das fixierende Polyurethan eine wiederkehrende Grundeinheit der allgemeinen Formel (I) aufweist,
-O-B-O-CO-NH-R-NH-CO- (I),
in der bedeuten:
- B eine zweiwertige C₁-C₃₀-Kohlenwasserstoffgruppe, die gegebenenfalls mit einer Gruppe substituiert ist, die eine oder mehrere Carbonsäurefunktionen und/oder eine oder mehrere Sulfonsäurefunktionen aufweist, wobei die Carbonsäurefunktionen und/oder die Sulfonsäurefunktionen in freier Form oder in teilweise oder vollständig mit einer anorganischen oder organischen Base neutralisierter Form vorliegen,
und
- R eine zweiwertige Gruppe, die ausgewählt ist unter Alkylengruppen vom aromatischen Typ, vom aliphatischen C₁-C₂₀-Typ und vom cycloaliphatischen C₁-C₂₀-Typ, wobei diese Gruppen gegebenenfalls substituiert sind.

6. Aerosolvorrichtung nach einem der Ansprüche 1 bis 5, **dadurch gekennzeichnet, dass** das fixierende Polyurethan eine wiederkehrende Grundeinheit der allgemeinen Formel (II) aufweist,
-O-P-O-CO-NH-R-NH-CO- (II),
in der bedeuten:
- P ein Polysiloxan-Segment
und
- R eine zweiwertige Gruppe, die ausgewählt ist unter Alkylengruppen vom aromatischen Typ, vom aliphatischen C₁-C₂₀-Typ und vom cycloaliphatischen C₁-C₂₀-Typ, wobei diese Gruppen gegebenenfalls substituiert sind.

7. Aerosolvorrichtung nach Anspruch 5 oder 6, **dadurch gekennzeichnet, dass** das fixierende Polyurethan ein Copolymer von Dimethylolpropionsäure, Isophorondiisocyanat, Neopentylglycol, Polyesterdiolen und siliconhaltigem Diamin ist.

8. Aerosolvorrichtung nach einem der vorhergehenden Ansprüche, **dadurch gekennzeichnet, dass** das fixierende Polyurethan in einer Menge von 0,5 bis 20 Gew.-% vorliegt, bezogen auf das Gesamtgewicht der Zusammensetzung.

9. Aerosolvorrichtung nach Anspruch 8, **dadurch gekennzeichnet, dass** das fixierende Polyurethan in einer Menge von 1 bis 12 Gew.-% vorliegt, bezogen auf das Gesamtgewicht der Zusammensetzung.

10. Vorrichtung nach einem der vorhergehenden Ansprüche, **dadurch gekennzeichnet, dass** das kosmetisch akzeptable alkoholische oder wässerig-alkoholische Medium aus mindestens einem Alkohol oder einem Gemisch von mindestens einem Alkohol mit Wasser besteht.

11. Aerosolvorrichtung nach Anspruch 10, **dadurch gekennzeichnet, dass** der Alkohol ein C₁-C₄-Alkohol ist.

12. Aerosolvorrichtung nach Anspruch 11, **dadurch gekennzeichnet, dass** der Alkohol Ethanol oder Isopropanol ist.

13. Aerosolvorrichtung nach einem der vorhergehenden Ansprüche, **dadurch gekennzeichnet, dass** die Frisierzusammensetzung ferner Zusatzstoffe aufweist, die ausgewählt sind unter Siliconen in löslicher, dispergierter und mikrodispergierter Form, nichtionischen, kationischen, anionischen oder amphoteren fixierenden Polymeren, die keine Polyurethane sind, Behandlungs-Wirkstoffen, Hydratisierungsmitteln, UV-Filtern, Säuren, Basen, Weichmachern, Solubilisierungsmitteln, Konservierungsmitteln, Vitaminen und Provitaminen, Färbemitteln, Pigmenten, anionischen, kationischen, nichtionischen oder amphoteren Tensiden, Parfums und Korrosionsschutzmitteln sowie Gemischen dieser Stoffe.

14. Aerosolvorrichtung nach einem der vorhergehenden Ansprüche, **dadurch gekennzeichnet, dass** es sich dabei um einen Haarlack handelt.

15. Frisierverfahren, **dadurch gekennzeichnet, dass** die in der Aerosolvorrichtung nach einem der vorhergehenden Ansprüche enthaltene Frisierzusammensetzung auf die feuchten oder trockenen Haare aufgesprüht wird.
